(19) **Europäisches Patentamt / European Patent Office / Office européen des brevets**

(11) **EP 4 381 290 B1**

(12) **EUROPÄISCHE PATENTSCHRIFT**

(45) Veröffentlichungstag und Bekanntmachung des Hinweises auf die Patenterteilung:
**29.01.2025 Patentblatt 2025/05**

(21) Anmeldenummer: **23723473.7**

(22) Anmeldetag: **27.04.2023**

(51) Internationale Patentklassifikation (IPC):
**G01N 33/28** (2006.01)    **C10G 75/00** (2006.01)

(52) Gemeinsame Patentklassifikation (CPC):
**G01N 33/2829; C10G 75/00**

(86) Internationale Anmeldenummer:
**PCT/EP2023/061117**

(87) Internationale Veröffentlichungsnummer:
**WO 2023/209094 (02.11.2023 Gazette 2023/44)**

(54) **VERFAHREN ZUR BESTIMMUNG EINER NORMIERTEN RUSSBILDUNGSNEIGUNG EINES STOFFGEMISCHS UND VON REPRÄSENTATIVEN INDIZES ZUR BERECHNUNG EINES GESAMT-RUSSBILDUNGSNEIGUNGS-INDEX**

METHOD FOR DETERMINING A STANDARDISED SOOTING TENDENCY OF A SUBSTANCE MIXTURE, AND FOR DETERMINING REPRESENTATIVE INDICES FOR CALCULATING A TOTAL SOOTING TENDENCY INDEX

PROCÉDÉ DE DÉTERMINATION D'UNE TENDANCE À LA SALISSURE NORMALISÉE D'UN MÉLANGE DE SUBSTANCES ET DE DÉTERMINATION D'INDICES REPRÉSENTATIFS POUR LE CALCUL D'UN INDICE DE TENDANCE À LA SALISSURE TOTAL

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **29.04.2022 DE 102022110522**

(43) Veröffentlichungstag der Anmeldung:
**12.06.2024 Patentblatt 2024/24**

(73) Patentinhaber: **VOLKSWAGEN AG**
**38440 Wolfsburg (DE)**

(72) Erfinder:
• **BITTNER, Yvonne**
**38102 Braunschweig (DE)**
• **KÖRTKE, Kerstin**
**30163 Hannover (DE)**

(74) Vertreter: **Gulde & Partner**
**Patent- und Rechtsanwaltskanzlei mbB**
**Berliner Freiheit 2**
**10785 Berlin (DE)**

(56) Entgegenhaltungen:
• **DAS DHRUBAJYOTI D ET AL: "Sooting tendencies of diesel fuels, jet fuels, and their surrogates in diffusion flames", FUEL, IPC SIENCE AND TECHNOLOGY PRESS , GUILDFORD, GB, vol. 197, 27 February 2017 (2017-02-27), pages 445 - 458, XP029957042, ISSN: 0016-2361, DOI: 10.1016/ J.FUEL.2017.01.099**
• **PEPIOT-DESJARDINS P ET AL: "Structural group analysis for soot reduction tendency of oxygenated fuels", COMBUSTION AND FLAME, ELSEVIER SCIENCE PUBLISHING CO., INC., NEW YORK, NY.; US, AMSTERDAM, NL, vol. 154, no. 1-2, 29 April 2008 (2008-04-29), pages 191 - 205, XP022697121, ISSN: 0010-2180, [retrieved on 20080429], DOI: 10.1016/ J.COMBUSTFLAME.2008.03.017**
• **AHMED QASEM MOHAMMED AMEEN ET AL: "A machine learning model for predicting threshold sooting index (TSI) of fuels containing alcohols and ethers", FUEL, IPC SIENCE AND TECHNOLOGY PRESS , GUILDFORD, GB, vol. 322, 13 April 2022 (2022-04-13), XP087087779, ISSN: 0016-2361, [retrieved on 20220413], DOI: 10.1016/J.FUEL.2022.123941**

- **KATHROTIA TRUPTI ET AL: "Predicting the soot emission tendency of real fuels - A relative assessment based on an empirical formula", FUEL, IPC SIENCE AND TECHNOLOGY PRESS , GUILDFORD, GB, vol. 261, 31 October 2019 (2019-10-31), XP085892248, ISSN: 0016-2361, [retrieved on 20191031], DOI: 10.1016/ J.FUEL.2019.116482**

**Beschreibung**

Die Erfindung betrifft ein Verfahren zur Bestimmung der Rußbildungsneigung mittels einer gaschromatographischen Komponentenanalyse von Kohlenwasserstofftypen und Oxygenaten eines Stoffgemischs.

Das Thema der Rußpartikelbildung von Kraftstoffen ist in der heutigen Zeit sehr entscheidend, da die Emissionen beziehungsweise die Rußpartikelbildung von Kraftfahrzeugen immer weiter gesenkt werden soll. Dies hängt insbesondere mit der Verschärfung der Richtlinien im Zusammenhang mit der EU-7-Reform zusammen. Dabei müssen für die Kraftfahrzeuge beziehungsweise für die Kraftstoffe die neuen Richtlinien erfüllt werden und eine Brandbreite von unterschiedlichen Kraftstoffen getestet werden. Des Weiteren muss die Bestandswirksamkeit zum Beispiel für City Fuels überprüft werden. Der YSI (Yield Sooting Index) stellt eine gute Grundlage zur Beschreibung der Rußbildungsneigung dar. Der YSI wird in deutscher Sprache als Rußbildungsneigungs-Index bezeichnet. Dieser Index ist momentan kompliziert zu bestimmen. Es besteht danach die Möglichkeit der Bestimmung des Rußbildungsneigungs-Index mittels einer Einzelkomponentenanalyse eines Kraftstoffes. In nachteiliger Weise muss somit bei der Bestimmung des Rußbildungsneigungs-Index eine Einzelkomponentenanalyse durchgeführt werden. Diese Analyse beinhaltet viele Daten und ist sehr zeitaufwendig und kostenintensiv. Des Weiteren besteht das Problem, dass bei dieser Methode nicht alle Komponenten zugeordnet werden können und als Unbekannte gelistet sind. Dies führt letztlich in nachteiliger Weise zu Abweichungen bei der Berechnung.

Aus dem Stand der Technik ist die Druckschrift DE 689 27 133 T2 bekannt. Sie schlägt ein Verfahren zur Bestimmung des Gehalts an aromatischem Kohlenstoff einer Kohlenwasserstoffe enthaltenden Lösung vor, die mehr als eine aromatische Verbindung enthält, bei dem wie folgt vorgegangen wird. (a) Die Lösung wird mittels Flüssigkeitschromatographie in Fraktionen getrennt. (b) Jede Fraktion wird separat mit UV-Licht in einem Wellenlängenbereich, von dem mindestens ein Teil im Bereich von 200 nm bis 400 nm liegt, bestrahlt. (c) Die Absorption des UV-Lichts wird durch die aromatischen Kohlenwasserstoffe in jeder Fraktion gemessen. (d) Anschließend wird das Integral der Absorption als Funktion der Photonenenergie über der Energie entsprechend dem Wellenlängenbereich ermittelt. (e) Schließlich wird der Gehalt an aromatischem Kohlenstoff aus dem Absorptionsintegral bestimmt.

Die Druckschrift EP 1 953 545 A1 offenbart ein Verfahren zur quantitativen Analyse eines Gemisches molekularer Verbindungen durch zweidimensionale Gaschromatographie, bei dem aus einem chromatographischen Signal (SB) ein zweidimensionales Chromatogramm (CHR) erzeugt wird, und chromatographische Peaks an der Polygonhilfe selektiert werden.

Das Verfahren umfasst die folgenden Schritte für mindestens ein Polygon: Anpassen eines Polygons, indem Teile des chromatographischen Signals, die in dem Polygon enthalten sind, identifiziert werden, mit den folgenden weiteren Schritten: (a) Bestimmen von Startzeiten, Endzeiten und maximalen chromatographischen Peaks, die auf den Portionen vorhanden sind. (b) Das Polygon wird durch Verschieben von Schnittpunkten zwischen dem Polygon und den Abschnitten als Funktion der Startzeiten, der Endzeiten und der maximalen chromatographischen Peaks eingestellt. (c) Eine Menge mindestens einer molekularen Verbindung wird bestimmt, indem die Fläche des so angepassten Polygons berechnet wird.

Die Druckschrift WO 1997/01142 A1 beschreibt ein Verfahren zum Vorhersagen von physikalischen, Wahrnehmungs-, Leistungs- oder chemischen Eigenschaften eines komplexen Kohlenwasserstoffgemisches, das Folgendes umfasst: (a) Auswählen mindestens einer Eigenschaft des Kohlenwasserstoffgemisches. (b) Auswählen von Referenzproben, wobei die Referenzproben charakteristische Verbindungstypen enthalten, die in dem komplexen Kohlenwasserstoffgemisch vorhanden sind und die bekannte Werte der in Schritt (a) ausgewählten Eigenschaft oder Eigenschaften aufweisen. (c) Erstellen eines Probesatzes durch die Schritte: (1) Injizieren jeder Referenzprobe in einen Gaschromatographen, der mit einem Massenspektrometer verbunden ist, wodurch zumindest eine teilweise Trennung des Kohlenwasserstoffgemisches in chemische Bestandteile bewirkt wird. (2) Einführen der chemischen Bestandteile jeder Referenzprobe in das Massenspektrometer unter dynamischen Strömungsbedingungen. (3) Erhalten einer Reihe von zeitaufgelösten Massenchromatogrammen für jede Referenzprobe. (4) Kalibrieren der Massenchromatogramme, um die Retentionszeiten zu korrigieren. (5) Auswählen einer Reihe von korrigierten Retentionszeitfenstern. (6) Auswählen einer Reihe von Molekül- und/oder Fragmentionen innerhalb jedes Retentionszeitfensters, wobei die Ionen repräsentativ für charakteristische Verbindungen oder Molekülklassen sind, die innerhalb des Retentionszeitfensters erwartet werden. (7) Aufzeichnen der Gesamtmenge jeder charakteristischen Verbindung oder Verbindungsgruppe, die in Schritt c (6) ausgewählt wurde. (8) Bilden der Daten aus den Schritten c (6) und c (7) in eine X-Block-Matrix. (9) Bilden der in (a) ausgewählten Daten für in (b) ausgewählte Referenzproben in eine Y-Block-Matrix. (10) Analysieren der Daten aus den Schritten c (8) und c (9) durch multivariate Korrelationstechniken einschließlich partieller kleinster Quadrate, Hauptkomponentenregression oder Ridge-Regression, um eine Reihe von Koeffizienten zu erzeugen. (d) Unterwerfen einer unbekannten Kohlenwasserstoffmischung den Schritten c (I) bis c (3) auf die gleiche Weise wie die Referenzprobe, um eine Reihe von zeitaufgelösten Massenchromatogrammen zu erzeugen. (e) Wiederholen der Schritte c (4) bis c (8) für jedes Massenchromatogramm von Schritt (d) und (f) Multiplizieren der Matrix aus Schritt (e) mit den Koeffizienten aus Schritt c (10), um einen vorhergesagten Wert der Eigenschaft oder Eigenschaften zu erzeugen.

DAS DHRUBAJYOTI D ET AL: "Sooting tendencies of diesel fuels, jet fuels, and their surrogates in diffusion flames", FUEL, Bd. 197, 27. Februar 2017, Seiten 445-458, XP029957042,ISSN: 0016-2361, DOI: 10.1016/J.FUEL.2017.01.099 offenbart ein Verfahren zur Bestimmung eines Gesamtrußbildungsneigungsindexes eines Kraftstoffs.

Zusammengefasst wird im Stand der Technik gemäß der Druckschrift DE 689 27 133 T2 eine (Molekül-) Gruppenbildung vorgeschlagen, während die Druckschrift EP 1 953 545 A1 die Bestimmung in Peakgruppen vorsieht, wobei schließlich die Druckschrift WO 1997/01142 A1 das Auswählen von Molekül- und/oder Fragmentionen offenbart, um Kohlenwasserstoffgemische zu analysieren.

Der Erfindung liegt die Aufgabe zugrunde, ein vereinfachtes Verfahren zur Bestimmung eines Rußbildungsneigungs-Index für ein Stoffgemisch zu entwickeln, welches aus einer Vielzahl von sich stofflich und hinsichtlich des Volumenanteils unterscheidenden Einzelkomponenten, insbesondere Kohlenwasserstofftypen und Oxygenaten, besteht, wobei das Verfahrensergebnis geeignet sein soll, die Rußbildungsneigung von zu vergleichenden Stoffgemischen in Form eines den Stoffgemischen zugeordneten Rußbildungsneigungs-Index in die Normung einzubringen.

Ausgangspunkt des erfindungsgemäßen Verfahrens ist die Norm ISO 22854:2016 mit dem Titel "Flüssige Mineralölerzeugnisse - Bestimmung von Kohlenwasserstofftypen und Oxygenaten in Kraftfahrzeugbenzin und in Ethanol (E85) - Mehrdimensionales Gaschromatographieverfahren". Für die meisten heutzutage auftretenden Kraftstoffe liegt eine Reformulyzeranalyse nach ISO 22854 vor. Es liegt somit zumeist keine Einzelkomponentenanalyse, sondern eine Reformulyzeranalyse nach der Norm ISO 22854 vor. Nach der ISO 22854 wird eine Gaschromatographie durchgeführt. Das Ergebnis wird anschließend als sogenannter Reformulyzer in einem sogenannten Reformulyzer-Output dargestellt. Bei der Reformulyzeranalyse nach ISO 22854 erfolgt eine Einteilung der Einzelkomponenten in funktionale Obergruppen und in Reformulyzer-Gruppen, wobei die Reformulyzeranalyse die Reformulyzer-Gruppen i enthält, die nach Anzahl der Kohlenstoffatome unterschieden werden.

Die nach Anzahl der Kohlenstoffatome eingeteilten Reformulyzer-Gruppen i werden auch als Reformulyzer-Untergruppen bezeichnet.

Bei der mehrdimensionalen Gaschromatographie wird zudem der prozentuale volumetrische Anteil $V_i$ der jeweiligen Reformulyzer-Untergruppe i bestimmt.

Zur Bestimmung der Rußbildungsneigung mittels einer gaschromatographischen Komponentenanalyse von Kohlenwasserstofftypen und Oxygenaten eines Stoffgemischs, insbesondere eines Kraftstoffs (F) oder eines Zusatzstoffs (B) mittels einer Reformulyzeranalyse sind folgende Schritte vorgesehen:

(I) Einteilen der innerhalb der Komponentenanalyse bestimmten Kohlenwasserstofftypen und Oxygenaten des Stoffgemischs in funktionale Reformulyzer-Obergruppen,

(II) Einteilen der bestimmten Kohlenwasserstofftypen und Oxygenaten nach der Anzahl der Kohlenstoffatome in Reformulyzer-Untergruppen (i), die den funktionalen Reformulyzer-Obergruppen zugeordnet sind,

(III) Bestimmen des prozentualen volumetrischen Anteils ($V_i$) der jeweiligen Reformulyzer-Untergruppe (i) vom Gesamtvolumen des Stoffgemischs. Erfindungsgemäß sind als weitere Schritte vorgesehen, das

(IV) Zuordnen eines Rußbildungsneigungs-Index ($YSI_i$) zu jeder Reformulyzer-Untergruppe (i) und das

(V) Bestimmen eines Gesamt-Rußbildungsneigungs-Index ($YSI_{gesamt}$) nach der Gleichung (1) $YSI_{Gesamt} = \Sigma\, YSI_i \cdot V_i / 100$, wobei der volumetrische Anteil ($V_i$) jeder Reformulyzer-Untergruppe (i) mit dem der Reformulyzer-Untergruppe (i) zugeordneten Rußbildungsneigungs-Index ($YSI_i$) multipliziert wird, wonach die ermittelten Werte zu dem Gesamt-Rußbildungsneigungs-Index ($YSI_{gesamt}$) aufsummiert werden.

Bevorzugte Ausgestaltungen des Verfahrens sind in den Unteransprüchen und in der Beschreibung erläutert.

Die Erfindung wird nachfolgend anhand der zugehörigen Zeichnungen erläutert. Es zeigen:

Figur 1          eine grundsätzliche Verfahrensweise in einer schematischen Übersichtsdarstellung und
Figuren 2 und 3     eine durchgeführte Reformulyzeranalyse nach ISO 22854.

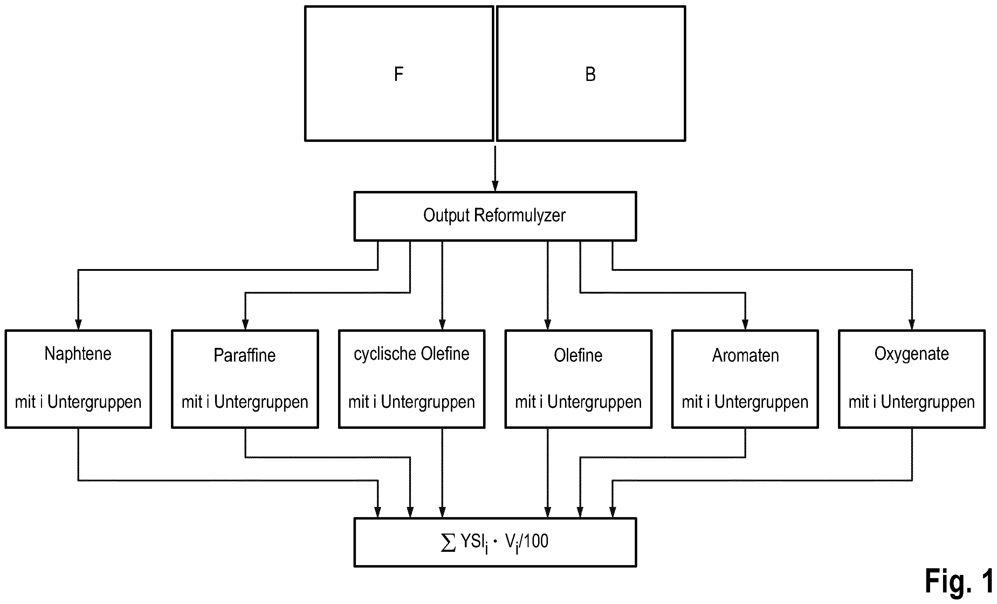

Figur 1 zeigt die grundsätzliche Verfahrensweise in einer schematischen Übersichtsdarstellung.

Es werden mittels des erfindungsgemäßen Verfahrens ottomotorische Kraftstoffe F oder auf der Basis von Kohlenwasserstoffen hergestellte Zusatzstoffe B, sogenannte Blends, die zumeist den ottomotorischen Kraftstoffen F als Additive zugeschlagen werden, auf ihre Rußbildungsneigung untersucht.

Innerhalb des Verfahrens liefert eine durchgeführte Reformulyzeranalyse nach ISO 22854, wie erläutert und in den Figuren 1 bis 3 verdeutlicht ist, eine Einteilung der Einzelkomponenten in funktionale Reformulyzer-Obergruppen und in Reformulyzer-Untergruppen i, wobei innerhalb der Reformulyzeranalyse nach ISO 22854 der prozentuale volumet-rische Anteil $V_i$ der jeweiligen Reformulyzer-Untergruppe i bestimmt wird und somit zu weiterer Ausgestaltung des Verfahrens vorliegt.

Die funktionalen Reformulyzer-Obergruppen sind Naphthene, Paraffine, cyclische Olefine, Olefine, Aromaten und Oxygenate.

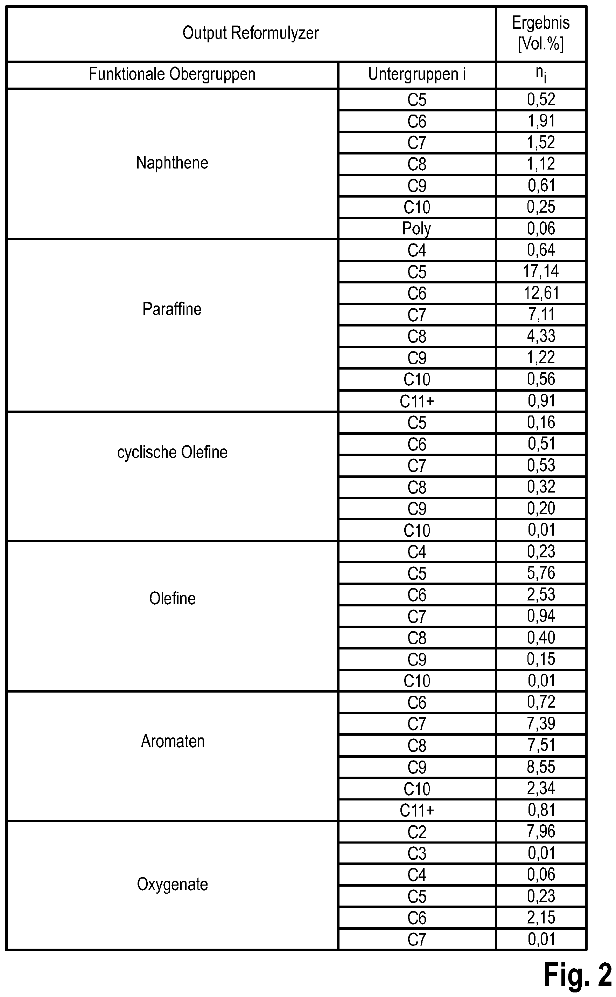

Die Reformulyzer-Untergruppen i sind gemäß Figur 2 den jeweiligen funktionalen Obergruppen zugeordnet und nach Anzahl mit dem Bezugszeichen $V_i$ (V = volumetrischer Anteil in % vom Gesamtvolumen und i = Anzahl der Kohlenstoffatome) der Kohlenstoffatome sortiert.

Erfindungsgemäß wird jeder dieser Reformulyzer-Untergruppen i ein Rußbildungsneigungs-Index zugeordnet, der nachfolgend als $YSI_i$ bezeichnet und mit dem Bezugszeichen $YSI_i$ versehen ist. Man könnte somit auch von einem Untergruppen-$YSI_i$ sprechen.

Bei der Reformulyzeranalyse nach ISO 22854 liegt zu den einzelnen Einzelkomponenten nur die Gruppeneinteilung i und der prozentuale volumetrische Anteil $V_i$ vor, nicht jedoch der Rußbildungsneigungs-Index der einzelnen Komponenten, so dass schließlich auch kein Wert für einen Rußbildungsneigungs-Index $YSI_i$ einer Reformulyzer-Untergruppen i gebildet werden kann.

Diesen Nachteil überwindet das erfindungsgemäße Verfahren, wie folgt:
Der Rußbildungsneigungs-Index $YSI_i$ der jeweiligen Reformulyzer-Untergruppe i wird aus einer dafür zur Verfügung stehenden Datenbank entnommen werden, welche die Anmelderin durch Auswertung der den jeweiligen Reformulyzer-Untergruppen i gehörenden Einzelkomponenten erstellt hat.

Mit anderen Worten, die Datenbank umfasst die zu einer Reformulyzer-Untergruppe i gehörenden Einzelkomponenten mit dem jeweiligen $YSI_i$ der jeweiligen Einzelkomponente, wobei innerhalb der Datenbank erfindungsgemäß die Ermittlung eines für die jeweilige Reformulyzer-Untergruppe i = $YSI_i$ vorgenommen wird.

Dieser, der jeweiligen Reformulyzer-Untergruppe i zugeordnete $YSI_i$ gilt datenbankseitig unabhängig von dem prozentualen volumetrischen Anteil $V_i$ von dem Gesamtvolumen des untersuchten Stoffgemischs, wobei der prozentuale volumetrische Anteil $V_i$ in vorteilhafter Weise durch die Reformulyzeranalyse nach ISO 22854 bereitgestellt wird.

Um den zu verwendenden $YSI_i$ festzulegen, werden unterschiedliche Verfahrensvarianten vorgeschlagen. Die nachfolgenden Verfahrensvarianten zur Ermittlung des $YSI_i$ repräsentieren den $YSI_i$ der Reformulyzer-Untergruppe i auf verschiedene Weise, wobei sich die Verfahrensvarianten insbesondere hinsichtlich des Aufwandes unterscheiden, der betrieben wird, um den $YSI_i$ der jeweiligen Reformulyzer-Untergruppe i zu ermitteln. Alle Vorgehensweise führen zum Erfolg, unterscheiden sich jedoch hinsichtlich der Datenbasis, die aus der Datenbank entnommen und zur Verfügung gestellt wird, wie nachfolgend anhand der Beschreibung der Verfahrensvariante verdeutlicht wird.

Grundsatz: Erfindungsgemäß wird jedem dieser Reformulyzer-Untergruppen i ein repräsentativer Rußbildungsneigungs-Index zugeordnet.

Je nach der Bildung des repräsentativen Rußbildungsneigungs-Index schlägt die Erfindung folgende bevorzugte Verfahrensvarianten vor:
Bildung eines repräsentativen Rußbildungsneigungs-Index $YSI_i$-M je Reformulyzer-Untergruppe i auf der Basis der aus der Datenbank entnommenen $YSI_i$-Werte der Einzelkomponenten der jeweiligen Reformulyzer-Untergruppe i in einer ersten Verfahrensvariante:
Aus eigenen DHA-Analysen zu den Einzelkomponenten der jeweiligen Reformulyzer-Untergruppen i und anmelderfremden Datenbanken wurden von der Anmelderin die $YSI_i$-Werte der jeweiligen Einzelkomponenten der jeweiligen Reformulyzer-Untergruppen i erfasst.

Beispielsweise beinhaltet eine Reformulyzer-Untergruppe i = C10 in der Reformulyzer-Obergruppe der Aromaten = 35 verschiedene Einzelkomponenten. Innerhalb der Reformulyzer-Untergruppe i = C10 wird jeder Einzelkomponente ein $YSI_i$-Wert zugeordnet.

Zur Vereinfachung des Verfahrens wird aus den datenbankseitigen $YSI_i$-Werten der Einzelkomponenten der Reformulyzer-Untergruppe i ein arithmetischer Mittelwert gebildet, welcher der Reformulyzer-Untergruppe i als repräsentativer $YSI_i$-M zugeordnet wird, der bei der nachfolgend erläuterten Berechnung des Gesamt-Rußbildungsneigungs-Index $YSI_{gesamt}$ als $YSI_i$ verwendet wird.

Bildung eines repräsentativen Rußbildungsneigungs-Index $YSI_i$-Mid je Reformulyzer-Untergruppe i auf der Basis der aus der Datenbank entnommenen $YSI_i$-Werte der Einzelkomponenten der jeweiligen Reformulyzer-Untergruppe i in einer zweiten Verfahrensvariante:
Die Ausgangslage hinsichtlich der zur Verfügung stehenden $YSI_i$-Werte aus eigenen DHA-Analysen zu den Einzelkomponenten der jeweiligen Reformulyzer-Untergruppen i und anmelderfremden Datenbanken entspricht der ersten Verfahrensvariante.

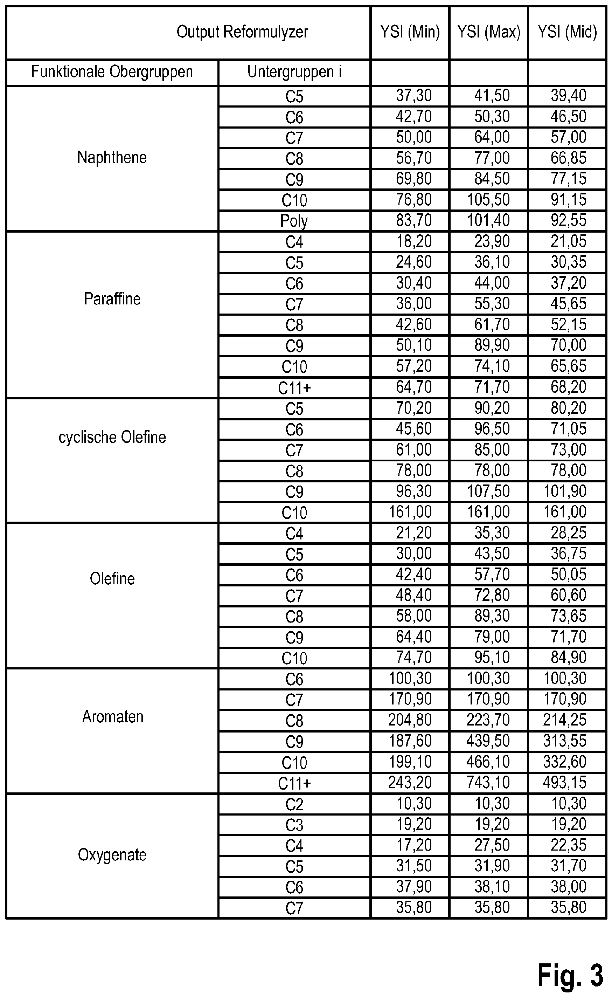

Im Unterschied zu der ersten Verfahrensvariante ist vorgesehen, dass der repräsentative Rußbildungsneigungs-Index ein mittlerer Rußbildungsneigungs-Index $YSI_i$-Mid (vergleiche vorab Figur 3) ist, der durch Berechnung des arithmetischen Mittelwertes zwischen einem minimalen $YSI_i$-Min-Wert und einem maximalen $YSI_i$-Max-Wertaller Einzelkomponenten einer Reformulyzer-Untergruppe i ermittelt wird. In der dritten Spalte der Figur 3 ist der zugehörige mittleren Rußbildungsneigungs-Index Mittelwert $YSI_i$-Mid verzeichnet, der bei der nachfolgend erläuterte Berechnung des Gesamt-Rußbildungsneigungs-Index $YSI_{gesamt}$ als $YSI_i$ verwendet wird.

So kann, wie in Figur 3 verdeutlicht ist, beispielsweise die Reformulyzer-Untergruppe i = C10 in der Reformulyzer-Obergruppe Aromaten = 35 verschiedene Einzelkomponenten beinhalten. Der $YSI_i$-Min-Wert beträgt = 199,1 und

der YSI$_i$-Max-Wert beträgt = 466,1, wodurch sich in diesem Ausführungsbeispiel der repräsentative mittlere Rußbildungs-neigungs-Index Mittelwert YSI$_i$ Mid für die Reformulyzer-Untergruppe i = C10 zu YSI$_i$ Mid = 332,60 ergibt.

Der mittlere Rußbildungsneigungs-Index YSI$_i$ Mid wird der Reformulyzer-Untergruppe i als repräsentativer YSI$_i$-Mid zugeordnet, der bei der nachfolgend erläuterten Berechnung des Gesamt-Rußbildungsneigungs-Index YSI$_{gesamt}$ als YSI$_i$ verwendet wird.

Bildung eines repräsentativen Rußbildungsneigungs-Index YSI$_i$-GewM je Reformulyzer-Untergruppe i auf der Basis der aus der Datenbank entnommenen YSI$_i$-Werte der Einzelkomponente der jeweiligen Reformulyzer-Untergruppe i in einer dritten Verfahrensvariante:

Die Ausgangslage hinsichtlich der zur Verfügung stehenden YSI$_i$-Werte aus eigenen DHA-Analysen zu den Einzelkomponenten der jeweiligen Reformulyzer-Untergruppen i und anmelderfremden Datenbanken entspricht der ersten und zweiten Verfahrensvariante.

Erfindungsgemäß ist in dieser dritten Verfahrensvariante ausgehend von der ersten Verfahrensvariante der arithmetischen Mittelwertbildung eine gewichtete arithmetische Mittelwertbildung für die einzelnen Reformulyzer-Untergruppen i vorgesehen.

Dazu wird/wurde in der Datenbank erfasst, wie häufig eine bestimmte Einzelkomponente in verschiedenen untersuchten Stoffgemischen, insbesondere Kraftstoffen F und Zusatzstoffen B, vorkommt.

Die Anmelderin verwendet eine große Datenbank mit den Einzelkomponenten zugeordneten Rußbildungsneigungs-Indizes aus mindestens 60 verschiedenen im Feld, das heißt sich im Einsatz befindenden üblichen Kraftstoffen F und Zusatzstoffen B, wodurch die Relevanz in Bezug auf die Häufigkeit der jeweiligen Einzelkomponente innerhalb einer Reformulyzer-Untergruppe i verifizierbar ist. Als häufig vorkommend und somit relevant werden die jeweiligen Einzelkomponenten gewertet, wenn sie in mindestens der Hälfte der betrachteten Kraftstoffe vorkommen.

Dadurch ist es in vorteilhafter Weise möglich, die Einzelkomponenten innerhalb einer Reformulyzer-Untergruppe i in Bezug auf ihre Häufigkeit zu wichten.

In einer funktionalen Reformulyzer-Obergruppe, beispielsweise den Aromaten, befinden sich beispielsweise 35 Einzelkomponenten.

Für die gewichtete arithmetische Mittelwertbildung werden den Einzelkomponenten die durch die Datenbankerfassung vorliegenden Häufigkeits-Werte zugeordnet und bei der Berechnung des repräsentativen gewichteten arith-metischen Mittelwertbildung YSI$_i$-GewM berücksichtigt.

Mit anderen Worten, selten vorkommende Einzelkomponenten, die in weniger als fünfzig Prozent der zuvor in der Datenbank ausgewerteten Kraftstoffe vorkommen, werden somit bei der Ermittlung des gewichteten arithmetischen Mittelwertes nicht gewichtet, so dass sich der repräsentative gewichtete mittlere arithmetische Rußbildungsneigungs-Index YSI$_i$-GewM von den anderen Rußbildungsneigungs-Indizes, dem YSI$_i$-M und dem YSI$_i$ Mid der ersten und zweiten Verfahrensvariante unterscheidet.

Gegenstand dieser Patentanmeldung ist somit das vereinfachte prinzipielle Verfahren zur Bestimmung einer normierten Rußbildungsneigung eines Kraftstoffs F oder eines Zusatzstoffs B beziehungsweise eines Gesamt-Rußbildungsneigungs-Index mit dem Bezugszeichen YSI$_{gesamt}$ für einen Kraftstoff F oder einen Zusatzstoff B und zudem die Art und Weise der Bestimmung des repräsentativen Rußbildungsneigungs-Index für die jeweiligen Reformulyzer-Untergruppen i.

Beispielhaft zeigt Figur 2 die volumetrischen Anteile V$_i$ der Reformulyzer-Untergruppen i, die zur Berechnung des Gesamt-Rußbildungsneigungs-Index mit dem Bezugszeichen YSI$_{gesamt}$ gemäß der Gleichung 1 herangezogen werden.

$$\text{(Gl. 1)} \qquad YSI_{Gesamt} = \sum YSI_i \cdot V_i / 100$$

| | |
|---|---|
| YSI$_{Gesamt}$ | → Gesamt-Rußbildungsneigungs-Index des Kraftstoffs F oder des Zusatzstoffes B |
| YSI$_i$ | → der YSI$_i$ der jeweiligen Untergruppe i |
| V$_i$ | → der ermittelte Volumenanteil V$_i$ in Prozent der jeweiligen Untergruppe i |

Gleichung 1 gibt die prinzipielle Lösung zur Gesamt-Rußbildungsneigungs-Index YSI$_{gesamt}$ wieder.

Je nach Verfahrensvariante wird als YSI$_i$ der jeweiligen Reformulyzer-Untergruppe i einer der zuvor erläuterten Indizes der YSI$_i$-M oder der YSI$_i$ Mid oder YSI$_i$-GewM berücksichtigt, das heißt in die Gleichung 1 eingesetzt.

Verschiedene Kraftstoffe F und/oder Zusatzstoffe B können somit in einfacherer Weise als bisher über den Gesamt-Rußbildungsneigungs-Index YSI$_{gesamt}$ miteinander verglichen werden, wobei ein höherer Wert des Gesamt-YSI einen mehr zu Rußbildung neigenden Kraftstoff F oder Zusatzstoff B kennzeichnet.

Die Vereinfachung dieser Vorgehensweise besteht insbesondere darin, dass für einen Kraftstoff F oder einen Zusatzstoff B keine herkömmliche aufwendige DHA-Auswertung (englisch: DHA = Detailed Hydrocarbon Analysis / deutsch: = Detaillierte Kohlenwasserstoffanalyse) aller Einzelkomponenten (Anzahl > 350) des jeweiligen Stoffgemischs, insbesondere eines Kraftstoffs F und/oder eines Zusatzstoffs B, mehr bestimmt werden muss.

In vorteilhafter Weise ist durch diese Vorgehensweise insbesondere keine Aufsummierung über alle > 350 Einzelkomponenten mehr notwendig, sondern es ist lediglich die Aufsummierung der Untergruppen i unter Berücksichtigung des prozentualen volumetrischen Anteils $V_i$ der jeweiligen Reformulyzer-Untergruppe i nötig.

Hinzu kommt, dass durch die Nutzung des Reformulyzers beziehungsweise des Outputs der Reformulyzeranalyse als Grundlage für die Auswertung in vorteilhafter Weise das Problem der vielen Unbekannten, wie oben erläutert, nicht mehr auftritt.

Letztlich ist dadurch eine robustere Bestimmung möglich, wobei das Ergebnis mit deutlich geringerem Aufwand zu erzielen ist, wie durch die vorherigen Beschreibung deutlich wird.

Das Verfahren weist bevorzugte Ausführungsvarianten auf.

In einer ersten Ausführungsvariante ist vorgesehen, dass zusätzlich noch funktionale Reformulyzer-Obergruppen zusammengefasst werden. So werden beispielsweise die funktionalen Reformulyzer-Obergruppen der Olefine und der cyclischen Olefine zu einer funktionalen Reformulyzer-Obergruppe zusammengefasst. Dadurch müssen im letzten Schritt des Verfahrens, dem Aufsummieren, weniger Daten aufsummiert werden.

In einer zweiten Ausführungsvariante ist vorgesehen, dass nahe beieinanderliegende Reformulyzer-Untergruppen i, deren Werte weniger als fünf Volumenprozent auseinanderliegen, innerhalb einer funktionalen Reformulyzer-Obergruppe zusammengefasst werden.

Beispielsweise können innerhalb einer der funktionalen Reformulyzer-Obergruppe die Reformulyzer-Untergruppen i, beispielsweise C7 und C8, zu einer einzigen Reformulyzer-Untergruppe zusammengefasst werden. Dadurch müssen im letzten Schritt des Verfahrens, dem Aufsummieren, weniger Daten aufsummiert werden.

In einer dritten Ausführungsvariante ist vorgesehen, dass nahe beieinanderliegende Reformulyzer-Untergruppen i, deren Werte weniger als fünf Volumenprozent auseinanderliegen, mehrerer funktionaler Reformulyzer-Obergruppen zusammengefasst werden. Somit können Reformulyzer-Untergruppen i über mehrere funktionale Reformulyzer-Obergruppen hinweg, beispielsweise C7 und C8 zu C(7+8) der Reformulyzer-Obergruppen zu einer Reformulyzer-Untergruppe i zusammengefasst werden. Dadurch müssen im letzten Schritt des Verfahrens, dem Aufsummieren, weniger Daten aufsummiert werden.

In einer vierten Ausführungsvariante ist vorgesehen, dass nahe beieinanderliegende Reformulyzer-Untergruppen i, deren Werte weniger als fünf Volumenprozent auseinanderliegen, aller funktionaler Reformulyzer-Obergruppen zusammengefasst werden. Beispielsweise kann über alle funktionalen Reformulyzer-Obergruppen hinweg, beispielsweise C7 und C8 zu C(7+8) aller Reformulyzer-Obergruppen zu einer Reformulyzer-Untergruppe i zusammengefasst werden. Dadurch müssen im letzten Schritt des Verfahrens, dem Aufsummieren, weniger Daten aufsummiert werden.

Die erste Ausführungsvariante kann in vorteilhafter Weise mit mindestens einer der zweiten bis vierten Ausführungsvariante kombiniert werden.

In vorteilhafter Weise verringert das "Zusammenfassen" gemäß den erläuterten Ausführungsvarianten den Aufwand der Berechnung des Gesamt-Rußbildungsneigungs-Index $YSI_{gesamt}$ in Abhängigkeit der gewählten Ausführungsvariante $YSI_{gesamt}$ entsprechend.

Die Vorteile des Verfahrens liegen zusammengefasst darin, dass die Kosten zur Bestimmung des Gesamt-Rußbildungsneigungs-Index $YSI_{gesamt}$ verringert werden, da keine Einzelkomponentenanalyse durchgeführt werden muss und bereits bestehende Standards (ISO 22854) genutzt werden können. Da bei Verwendung der Ergebnisse der Reformulyzeranalyse keine Unbekannten mehr auftreten, wird ferner als Vorteil des Verfahrens ein deutlich robusteres Ergebnis erzielt, welches nicht an unbekannten Eingangsgrößen scheitert, so dass das Ergebnis stets bestimmbar ist. Eine standardmäßige Ermittlung des Gesamt-YSI-Index $YSI_{gesamt}$ führt zudem verfahrensgemäß in vorteilhafter Weise zu einer standardmäßigen und normierbaren Aussage zur Rußbildungsneigung für jeden Kraftstoff F oder Zusatzstoff B. Das heißt, mittels des Gesamt-Rußbildungsneigungs-Index $YSI_{gesamt}$ kann in vorteilhafter Weise ein Vergleich der Gesamt-Rußbildungsneigungs-Indizies $YSI_{gesamt}$ verschiedener Kraftstoffe F und Zusatzstoffe B stattfinden und in einer Norm normiert werden.

Schließlich ist erfindungsgemäß vorgesehen, dass alle Kraftstoffe, bei denen die Rußbildungsneigung hervorgesagt werden muss, mittels dieses Verfahrens untersucht und bewertet, insbesondere normiert werden können. In vorteilhafter Weise kann das Verfahren bereits bei der Neuentwicklung von Kraftstoffen F oder Zusatzstoffen B als auch bei der Überprüfung von Bestandskraftstoffen angewendet werden.

Beim Betrieb der Fahrzeuge ist es erfindungsgemäß möglich, dass der Gesamt-Rußbildungsneigungs-Index $YSI_{gesamt}$ entweder automatisch (beispielsweise drahtlos) oder manuell an eine Empfangseinheit, insbesondere ein Steuergerät mit der Empfangseinheit eines Fahrzeug übermittelt wird. Liegt ein solcher Wert im Motor-Steuergerät vor, kann der Rußbildungsneigungs-Index $YSI_{gesamt}$ für eine präzisere Bestimmung der Steuerung der Betriebsparameter der Brennkraftmaschine und/oder der Abgasanlage genutzt werden. Der Rußbildungsneigungs-Index $YSI_{gesamt}$ kann dem Nutzer auch über das HMI angezeigt werden, so dass dem Nutzer eine normierte Information darüber vorliegt, ob er einen Kraftstoff F oder einen Zusatzstoff B nutzt, der zu einer hohen Rußbildung oder einer geringen Rußbildung neigt.

**Patentansprüche**

1. Verfahren zur Bestimmung der Rußbildungsneigung mittels einer gaschromatographischen Komponentenanalyse von Kohlenwasserstofftypen und Oxygenaten eines Stoffgemischs, insbesondere eines Kraftstoffs (F) oder eines Zusatzstoffs (B) mittels einer Reformulyzeranalyse mit den Schritten:

   (I) Einteilen der innerhalb der Komponentenanalyse bestimmten Kohlenwasserstofftypen und Oxygenaten des Stoffgemischs in funktionale Reformulyzer-Obergruppen,
   (II) Einteilen der bestimmten Kohlenwasserstofftypen und Oxygenaten nach der Anzahl der Kohlenstoffatome in Reformulyzer-Untergruppen (i), die den funktionalen Reformulyzer-Obergruppen zugeordnet sind,
   (III) Bestimmen des prozentualen volumetrischen Anteils ($V_i$) der jeweiligen Reformulyzer-Untergruppe (i) vom Gesamtvolumen des Stoffgemischs, **gekennzeichnet durch** die Schritte:
   (IV) Zuordnen eines Rußbildungsneigungs-Index ($YSI_i$) zu jeder Reformulyzer-Untergruppe (i),
   (V) Bestimmen eines Gesamt-Rußbildungsneigungs-Index ($YSI_{gesamt}$) nach der Gleichung (1) $YSI_{Gesamt} = \Sigma\ YSI_i \cdot V_i / 100$, wobei der volumetrische Anteil ($V_i$) jeder Reformulyzer-Untergruppe (i) mit dem der Reformulyzer-Untergruppe (i) zugeordneten Rußbildungsneigungs-Index ($YSI_i$) multipliziert wird, wonach die ermittelten Werte zu dem Gesamt-Rußbildungsneigungs-Index ($YSI_{gesamt}$) aufsummiert werden.

2. Verfahren nach Anspruch 1, **dadurch gekennzeichnet, dass** der zu der jeweiligen Reformulyzer-Untergruppe (i) zugeordnete Rußbildungsneigungs-Index ($YSI_i$) aus einer Datenbank entnommen wird, wobei der Wert des Rußbildungsneigungs-Index ($YSI_i$) der jeweiligen Reformulyzer-Untergruppe (i) auf der Basis der in der Datenbank vorliegenden jeweiligen Einzelkomponenten einer Reformulyzer-Untergruppe (i) bereitgestellt wird.

3. Verfahren nach Anspruch 2, **dadurch gekennzeichnet, dass** der Rußbildungsneigungs-Index ($YSI_i$) aus den datenbankseitigen $YSI_i$-Werten der Einzelkomponenten der Reformulyzer-Untergruppe (i) als arithmetischer Mittelwert gebildet wird, wobei in den jeweiligen arithmetischen Mittelwert entweder

   • die Werte der Rußbildungsneigungs-Indizes ($YSI_i$) aller Einzelkomponenten der Reformulyzer-Untergruppe (i) in einen repräsentativen arithmetischen Mittelwert ($YSI_i$-M) einfließen, oder
   • der minimale $YSI_i$-Wert ($YSI_i$-Min) und der maximale $YSI_i$-Wert ($YSI_i$-Max) der Rußbildungsneigungs-Indizes ($YSI_i$) aller Einzelkomponenten der Reformulyzer-Untergruppe (i) in einen repräsentativen arithmetischen Mittelwert ($YSI_i$-Mid) einfließen, oder
   • die Werte der Rußbildungsneigungs-Indizes ($YSI_i$) aller Einzelkomponenten der Reformulyzer-Untergruppe (i) in einen repräsentativen arithmetischen Mittelwert ($YSI_i$-GewM) einfließen, der die Häufigkeit des Auftretens der Einzelkomponenten innerhalb der Reformulyzer-Untergruppe (i) wichtet, wobei bei der Wichtung nur Einzelkomponenten berücksichtigt werden, die in mindestens fünfzig Prozent der in der Datenbank betrachteten Kraftstoffe vorkommen.

4. Verfahren nach Anspruch 1, **dadurch gekennzeichnet, dass** vor dem Bestimmen des Gesamt-Rußbildungsneigungs-Index ($YSI_{gesamt}$) mindestens zwei oder mehr funktionale Reformulyzer-Obergruppen zu einer funktionalen Reformulyzer-Obergruppe zusammengefasst werden.

5. Verfahren nach Anspruch 1, **dadurch gekennzeichnet, dass** vor dem Bestimmen des Gesamt-Rußbildungsneigungs-Index ($YSI_{gesamt}$) nahe beieinanderliegende Reformulyzer-Untergruppen (i) innerhalb einer funktionalen Reformulyzer-Obergruppe, deren Werte weniger als fünf Volumenprozent auseinanderliegen, zusammengefasst werden.

6. Verfahren nach Anspruch 1, **dadurch gekennzeichnet, dass** vor dem Bestimmen des Gesamt-Rußbildungsneigungs-Index ($YSI_{gesamt}$) nahe beieinanderliegende Reformulyzer-Untergruppen (i) mehrerer funktionaler Reformulyzer-Obergruppen, deren Werte weniger als fünf Volumenprozent auseinanderliegen, zusammengefasst werden.

7. Verfahren nach Anspruch 1, **dadurch gekennzeichnet, dass** vor dem Bestimmen des Gesamt-Rußbildungsneigungs-Index ($YSI_{gesamt}$) nahe beieinanderliegende Reformulyzer-Untergruppen (i) aller funktionaler Reformulyzer-Obergruppen, deren Werte weniger als fünf Volumenprozent auseinanderliegen, zusammengefasst werden.

8. Verfahren nach Anspruch 4, **dadurch gekennzeichnet, dass** das Zusammenfassen von funktionalen Reformulyzer-Obergruppen zu einer funktionalen Reformulyzer-Obergruppe mit dem Zusammenfassen von nahe beieinanderliegenden Reformulyzer-Untergruppen (i), deren Werte weniger als fünf Volumenprozent auseinanderliegen, nach mindestens einem der Ansprüche 5 bis 7 kombiniert wird

9. Verfahren nach Anspruch 1, **dadurch gekennzeichnet, dass** der Rußbildungsneigungs-Index ($YSI_{gesamt}$) entweder automatisch oder manuell an eine Empfangseinheit eines Fahrzeug übermittelt wird.

10. Verfahren nach Anspruch 9, **dadurch gekennzeichnet, dass** der Rußbildungsneigungs-Index ($YSI_{gesamt}$) als zusätzliche Eingangsgröße für eine präzisere Bestimmung der Steuerung der Betriebsparameter einer Brennkraftmaschine und/oder einer Abgasanlage des Fahrzeuges genutzt wird.

11. Verfahren nach Anspruch 1, **dadurch gekennzeichnet, dass** der Gesamt-Rußbildungsneigungs-Index ($YSI_{gesamt}$) zur Normierung hinsichtlich der Rußbildungsneigung von mehreren, mindestens zwei innerhalb des Verfahrens untersuchten unterschiedlichen Stoffgemischen, insbesondere Kraftstoffen (F) oder Zusatzstoffen (B), verwendet wird.

**Claims**

1. Method for determining yield sooting tendency using gas-chromatographic component analysis of hydrocarbon types and oxygenates of a substance mixture, in particular of a fuel (F) or of an additive (B), by means of a reformulyzer analysis, the method comprising the following steps:

   (I) classifying the hydrocarbon types and oxygenates of the substance mixture determined within the component analysis into functional reformulyzer supergroups,
   (II) classifying the determined hydrocarbon types and oxygenates according to the number of carbon atoms into reformulyzer subgroups (i), which are associated with the functional reformulyzer supergroups,
   (III) determining the percentage volumetric proportion ($V_i$) of the relevant reformulyzer subgroup (i) from the total volume of the substance mixture,
   **characterized by** the steps of:
   (IV) assigning a yield sooting index ($YSI_i$) to each reformulyzer subgroup (i),
   (V) determining a total yield sooting index ($YSI_{gesamt}$) according to the equation (1) $YSI_{Gesamt} = \Sigma\, YSI_i \cdot V_i/100$, wherein the volumetric proportion ($V_i$) of each reformulyzer subgroup (i) is multiplied by the yield sooting index ($YSI_i$) associated with the reformulyzer subgroup (i), after which the determined values are summed to form the total yield sooting index ($YSI_{gesamt}$).

2. Method according to claim 1, **characterized in that** the yield sooting index ($YSI_i$) associated with the relevant reformulyzer subgroup (i) is taken from a database, wherein the value of the yield sooting index ($YSI_i$) of the relevant reformulyzer subgroup (i) is provided on the basis of the respective individual components of a reformulyzer subgroup (i) present in the database.

3. Method according to claim 2, **characterized in that** the yield sooting index ($YSI_i$) is formed from the database $YSI_i$ values of the individual components of the reformulyzer subgroup (i) as an arithmetic mean, wherein, in the relevant arithmetic mean, either

   • the values of the yield sooting indices ($YSI_i$) of all individual components of the reformulyzer subgroup (i) flow into a representative arithmetic mean value ($YSI_i$-M), or
   • the minimum $YSI_i$ value ($YSI_i$-Min) and the maximum $YSI_i$ value ($YSI_i$-Max) of the yield sooting indices ($YSI_i$) of all individual components of the reformulyzer subgroup (i) flow into a representative arithmetic mean value ($YSI_i$-Mid), or
   • the values of the yield sooting indices ($YSI_i$) of all individual components of the reformulyzer subgroup (i) flow into a representative arithmetic mean value ($YSI_i$-GewM), which weighs the frequency of occurrence of the individual components within the reformulyzer subgroup (i), wherein only individual components which appear in at least fifty percent of the fuels considered in the database are taken into account during the weighting.

4. Method according to claim 1, **characterized in that,** prior to determining the total yield sooting index ($YSI_{gesamt}$), at least two or more functional reformulyzer supergroups are merged to form a functional reformulyzer supergroup.

**5.** Method according to claim 1, **characterized in that,** prior to determining the total yield sooting index (YSI$_{gesamt}$), reformulyzer subgroups (i) that are close to one another within a functional reformulyzer supergroup, the values of which are less than five volume percent apart, are merged.

**6.** Method according to claim 1, **characterized in that,** prior to determining the total yield sooting index (YSI$_{gesamt}$), reformulyzer subgroups (i) that are close to one another of a plurality of functional reformulyzer supergroups, the values of which are less than five volume percent apart, are merged.

**7.** Method according to claim 1, **characterized in that,** prior to determining the total yield sooting index (YSI$_{gesamt}$), reformulyzer subgroups (i) that are close to one another of all functional reformulyzer supergroups, the values of which are less than five volume percent apart, are merged.

**8.** Method according to claim 4, **characterized in that** merging functional reformulyzer supergroups to form a functional reformulyzer supergroup is combined with merging reformulyzer subgroups (i) that are close to one another, the values of which are less than five volume percent apart, according to at least one of claims 5 to 7.

**9.** Method according to claim 1, **characterized in that** the yield sooting index (YSI$_{gesamt}$) is transmitted either automatically or manually to a receiving unit of a vehicle.

**10.** Method according to claim 9, **characterized in that** the yield sooting index (YSI$_{gesamt}$) is used as an additional input variable for more precise determination of the control of the operating parameters of an internal combustion engine and/or an exhaust system of the vehicle.

**11.** Method according to claim 1, **characterized in that** the total yield sooting index (YSI$_{gesamt}$) is used for standardization with regard to the yield sooting tendency of a plurality of, at least two, different substance mixtures investigated within the method, in particular fuels (F) or additives (B).

**Revendications**

**1.** Procédé permettant la détermination de la tendance à la formation de suie à l'aide d'une analyse de composants par chromatographie en phase gazeuse de types d'hydrocarbures et d'oxygénats d'un mélange de substances, en particulier d'un carburant (F) ou d'un additif (B) à l'aide d'une analyse au reformeur, comportant les étapes consistant à:

(I) classer les types d'hydrocarbures et les oxygénats du mélange de substances, déterminés dans le cadre de l'analyse des composants, en groupes fonctionnels supérieurs de reformeur,
(II) classer les types d'hydrocarbures et les oxygénats déterminés en fonction du nombre d'atomes de carbone dans des sous-groupes de reformeur (i) associés aux groupes fonctionnels supérieurs de reformeur,
(III) déterminer le pourcentage de la fraction volumétrique ($V_i$) du sous-groupe de reformeur (i) respectif par rapport au volume total du mélange de substances,
**caractérisé par** les étapes suivantes :
(IV) attribution d'un indice de tendance à la formation de suie (YSI$_i$) à chaque sous-groupe de reformeur (i),
(V) détermination d'un indice total de tendance à la formation de suie (YSI$_{total}$) selon l'équation (1) YSI$_{total}$ = Σ YSI$_i$ · $V_i$/100, dans lequel la fraction volumétrique ($V_i$) de chaque sous-groupe de reformeur (i) est multipliée par l'indice de tendance à la formation de suie (YSI$_i$) associé au sous-groupe de reformeur (i), après quoi les valeurs obtenues sont additionnées pour former l'indice total de tendance à la formation de suie (YSI$_{total}$).

**2.** Procédé selon la revendication 1, **caractérisé en ce que** l'indice de tendance à la formation de suie (YSI$_i$) associé au sous-groupe de reformeur (i) respectif est prélevé dans une base de données, dans lequel la valeur de l'indice de tendance à la formation de suie (YSI$_i$) du sous-groupe de reformeur (i) respectif est fournie sur la base des composants individuels respectifs d'un sous-groupe de reformeur (i) présents dans la base de données.

**3.** Procédé selon la revendication 2, **caractérisé en ce que** l'indice de propension à la formation de suie (YSI$_i$) est formé en tant que valeur moyenne arithmétique à partir des valeurs YSI$_i$ côté base de données des composants individuels du sous-groupe de reformeur (i), dans lequel dans la valeur moyenne arithmétique respective soit

• les valeurs des indices de tendance à la formation de suie (YSI$_i$) de tous les composants individuels du sous-groupe de reformeur (i) sont intégrées dans une valeur moyenne arithmétique représentative (YSI$_i$-M), soit
• la valeur minimale YSI$_i$ (YSI$_i$-Min) et la valeur maximale YSI$_i$ (YSI$_i$-Max) des indices de tendance à la formation de suie (YSI$_i$) de tous les composants individuels du sous-groupe de reformeur (i) sont intégrées dans une valeur moyenne arithmétique représentative (YSI$_i$-Mid), soit
• les valeurs des indices de tendance à la formation de suie (YSI$_i$) de tous les composants individuels du sous-groupe de reformeur (i) sont intégrées dans une valeur moyenne arithmétique représentative (YSI$_i$-GewM) qui pondère la fréquence d'apparition des composants individuels au sein du sous-groupe de reformeur (i), dans lequel seuls les composants individuels présents dans au moins cinquante pour cent des carburants considérés dans la base de données sont pris en compte lors de la pondération.

4.  Procédé selon la revendication 1, **caractérisé en ce que,** avant la détermination de l'indice total de tendance à la formation de suie (YSI$_{total}$), au moins deux groupes fonctionnels supérieurs de reformeur ou plus sont regroupés en un groupe fonctionnel supérieur de reformeur.

5.  Procédé selon la revendication 1, **caractérisé en ce que,** avant la détermination de l'indice total de tendance à la formation de suie (YSI$_{total}$), des sous-groupes de reformeur (i) proches les uns des autres au sein d'un groupe fonctionnel supérieur de reformeur, dont les valeurs sont séparées par moins de cinq pour cent en volume, sont regroupés.

6.  Procédé selon la revendication 1, **caractérisé en ce que,** avant la détermination de l'indice total de tendance à la formation de suie (YSI$_{total}$), des sous-groupes de reformeur (i) proches les uns des autres de plusieurs groupes fonctionnels supérieurs de reformeur, dont les valeurs sont séparées par moins de cinq pour cent en volume, sont regroupés.

7.  Procédé selon la revendication 1, **caractérisé en ce que,** avant la détermination de l'indice total de tendance à la formation de suie (YSI$_{total}$), des sous-groupes de reformeur (i) proches les uns des autres de tous les groupes fonctionnels supérieurs de reformeur, dont les valeurs sont séparées par moins de cinq pour cent en volume, sont regroupés.

8.  Procédé selon la revendication 4, **caractérisé en ce que** le regroupement de groupes supérieurs fonctionnels de reformeur en un groupe supérieur fonctionnel de reformeur est combiné avec le regroupement de sous-groupes de reformeur (i) proches les uns des autres, dont les valeurs sont séparées par moins de cinq pour cent en volume, selon au moins l'une des revendications 5 à 7

9.  Procédé selon la revendication 1, **caractérisé en ce que** l'indice de tendance à la formation de suie (YSI$_{total}$) est transmis soit automatiquement soit manuellement à une unité de réception d'un véhicule.

10. Procédé selon la revendication 9, **caractérisé en ce que** l'indice de tendance à la formation de suie (YSI$_{total}$) est utilisé comme grandeur d'entrée supplémentaire pour une détermination plus précise de la commande des paramètres de fonctionnement d'un moteur à combustion interne et/ou d'un système d'échappement du véhicule.

11. Procédé selon la revendication 1, **caractérisé en ce que** l'indice total de tendance à la formation de suie (YSI$_{total}$) est utilisé pour la normalisation de la tendance à la formation de suie de plusieurs, au moins deux, mélanges de substances différents, en particulier de carburants (F) ou d'additifs (B), analysés au sein du procédé.

**Fig. 1**

| Output Reformulyzer | | Ergebnis [Vol.%] |
|---|---|---|
| Funktionale Obergruppen | Untergruppen i | $n_i$ |
| Naphthene | C5 | 0,52 |
| | C6 | 1,91 |
| | C7 | 1,52 |
| | C8 | 1,12 |
| | C9 | 0,61 |
| | C10 | 0,25 |
| | Poly | 0,06 |
| Paraffine | C4 | 0,64 |
| | C5 | 17,14 |
| | C6 | 12,61 |
| | C7 | 7,11 |
| | C8 | 4,33 |
| | C9 | 1,22 |
| | C10 | 0,56 |
| | C11+ | 0,91 |
| cyclische Olefine | C5 | 0,16 |
| | C6 | 0,51 |
| | C7 | 0,53 |
| | C8 | 0,32 |
| | C9 | 0,20 |
| | C10 | 0,01 |
| Olefine | C4 | 0,23 |
| | C5 | 5,76 |
| | C6 | 2,53 |
| | C7 | 0,94 |
| | C8 | 0,40 |
| | C9 | 0,15 |
| | C10 | 0,01 |
| Aromaten | C6 | 0,72 |
| | C7 | 7,39 |
| | C8 | 7,51 |
| | C9 | 8,55 |
| | C10 | 2,34 |
| | C11+ | 0,81 |
| Oxygenate | C2 | 7,96 |
| | C3 | 0,01 |
| | C4 | 0,06 |
| | C5 | 0,23 |
| | C6 | 2,15 |
| | C7 | 0,01 |

Fig. 2

| Output Reformulyzer | | YSI (Min) | YSI (Max) | YSI (Mid) |
|---|---|---|---|---|
| Funktionale Obergruppen | Untergruppen i | | | |
| Naphthene | C5 | 37,30 | 41,50 | 39,40 |
| | C6 | 42,70 | 50,30 | 46,50 |
| | C7 | 50,00 | 64,00 | 57,00 |
| | C8 | 56,70 | 77,00 | 66,85 |
| | C9 | 69,80 | 84,50 | 77,15 |
| | C10 | 76,80 | 105,50 | 91,15 |
| | Poly | 83,70 | 101,40 | 92,55 |
| Paraffine | C4 | 18,20 | 23,90 | 21,05 |
| | C5 | 24,60 | 36,10 | 30,35 |
| | C6 | 30,40 | 44,00 | 37,20 |
| | C7 | 36,00 | 55,30 | 45,65 |
| | C8 | 42,60 | 61,70 | 52,15 |
| | C9 | 50,10 | 89,90 | 70,00 |
| | C10 | 57,20 | 74,10 | 65,65 |
| | C11+ | 64,70 | 71,70 | 68,20 |
| cyclische Olefine | C5 | 70,20 | 90,20 | 80,20 |
| | C6 | 45,60 | 96,50 | 71,05 |
| | C7 | 61,00 | 85,00 | 73,00 |
| | C8 | 78,00 | 78,00 | 78,00 |
| | C9 | 96,30 | 107,50 | 101,90 |
| | C10 | 161,00 | 161,00 | 161,00 |
| Olefine | C4 | 21,20 | 35,30 | 28,25 |
| | C5 | 30,00 | 43,50 | 36,75 |
| | C6 | 42,40 | 57,70 | 50,05 |
| | C7 | 48,40 | 72,80 | 60,60 |
| | C8 | 58,00 | 89,30 | 73,65 |
| | C9 | 64,40 | 79,00 | 71,70 |
| | C10 | 74,70 | 95,10 | 84,90 |
| Aromaten | C6 | 100,30 | 100,30 | 100,30 |
| | C7 | 170,90 | 170,90 | 170,90 |
| | C8 | 204,80 | 223,70 | 214,25 |
| | C9 | 187,60 | 439,50 | 313,55 |
| | C10 | 199,10 | 466,10 | 332,60 |
| | C11+ | 243,20 | 743,10 | 493,15 |
| Oxygenate | C2 | 10,30 | 10,30 | 10,30 |
| | C3 | 19,20 | 19,20 | 19,20 |
| | C4 | 17,20 | 27,50 | 22,35 |
| | C5 | 31,50 | 31,90 | 31,70 |
| | C6 | 37,90 | 38,10 | 38,00 |
| | C7 | 35,80 | 35,80 | 35,80 |

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 68927133 T2
- EP 1953545 A1
- WO 199701142 A1

**In der Beschreibung aufgeführte Nicht-Patentliteratur**

- **DAS DHRUBAJYOTI D et al.** Sooting tendencies of diesel fuels, jet fuels, and their surrogates in diffusion flames. *FUEL*, 27 February 2017, vol. 197, ISSN 0016-2361, 445-458